# EUROPÄISCHE PATENTSCHRIFT

(11) **EP 1 579 817 B1**
(45) Veröffentlichungstag und Bekanntmachung des Hinweises auf die Patenterteilung: **27.02.2008**
(21) Anmeldenummer: 05006197.7
(22) Anmeldetag: 22.03.2005
(51) Int. Cl.: A61B 17/86, A61B 17/88

(54) **Schraubendreher für Knochenschrauben**
Screwdriver for bone screws
Tournevis pour vis à os

(30) Priorität: 27.03.2004 DE 202004004844 U
(43) Veröffentlichungstag der Anmeldung: 28.09.2005
(73) Patentinhaber: Richard Martin Medizintechnik GmbH, 78532 Tuttlingen (DE)
(72) Erfinder: Kreidler, Winfried, 78532 Tuttlingen (DE)
(74) Vertreter: Neymeyer, Franz

(56) Entgegenhaltungen:
- DE-C1- 19 806 662
- GB-A- 383 396
- US-A- 1 742 278
- US-A- 2 015 878
- US-A- 2 329 398
- US-A- 4 779 494
- US-A- 5 660 091
- US-A1- 2001 004 694

## Beschreibung

Die Erfindung betrifft einen Schraubendreher, der ein Werkzeug aufweist, das mit wenigstens drei sich radial und axial erstreckenden Werkzeugklingen versehen ist, welche mit nutenartigen Klingenschlitzen der Knochenschraube formschlüssig in Eingriff bringbar sind.

Knochenschrauben werden beispielsweise für die Osteosynthese entweder in Verbindung mit Knochenplatten oder auch einzeln verwendet. Insbesondere Kleinfragmentschrauben, auch sogenannte Corticalis-Schrauben kommen nach Schädelverletzungen nach Unfällen sowie bei gesichts- und kieferorthopädischen Operationen in der Handchirurgie und in unterschiedlichen Dimensionen auch in anderen Bereichen zum Einsatz. Solche Kleinfragmentschrauben sind auf Grund ihrer geringen Dimensionen äußerst schwierig zu handhaben. Die Länge solcher Kleinfragmentschrauben liegt etwa im Bereich zwischen 8 und 10 mm, wobei der Schraubenkopf einen maximalen Durchmesser von etwa 3 - 4 mm und der Schraubenschaft einen Durchmesser von 1,5 - 2,5 mm aufweisen kann. Es ist leicht vorstellbar, dass solche Kleinfragmentschrauben mittels eines Schraubendrehers nur äußerst schwierig handhabbar sind, da das Greifen von Hand und gleichzeitig das Ansetzen des Schraubendrehers und das Eindrehen solcher Kleinfragmentschrauben nur schwierig zu bewerkstelligen ist.

Um nun solche Kleinfragmentschrauben in einfacher Weise am Einsatzort handhaben zu können, sind verschiedene Systeme bekannt geworden, durch welche die Handhabung solcher Kleinfragmentschrauben vereinfacht werden soll. So sind beispielsweise aus der US 5,649,931, der DE 38 04 749 A1, der DE 100 44 714 C2 und der DE 35 39 502 C1 Systeme bekannt geworden, welche sich durch eine besondere Ausgestaltung des Schraubendrehers dadurch auszeichnen, dass die Kleinfragmentschrauben mittels Greifelementen am Schraubenkopf derart ergriffen werden können, dass die Kleinfragmentschraube bzw. Knochenschraube von den Werkzeugklingen des Schraubendrehers nicht mehr abrutschen kann.

So ist beispielsweise beim Gegenstand der DE 35 39 502 C1, ein Schraubendreher vorgesehen, welcher mit einem an einem Schaft sitzenden Werkzeug versehen ist, das mehrere Werkzeugklingen aufweist, mit welchen das Werkzeug formschlüssig mit entsprechenden Klingenschlitzen des Schraubenkopfes der Knochenschraube in Eingriff bringbar ist. Weiter ist an diesem Schaft eine Schraubenentnahmeeinrichtung vorgesehen, die ein Greiforgan aufweist, mittels welchem ein Schraubenkopf ergriffen werden kann. Dieses Greiforgan wird mittels einer längs des Schaftes verschiebbaren Spannhülse betätigt, wobei bei zurückgezogener Spannhülse die Greiforgane federelastisch radial nach außen vom Schaft abstehen. Beim Verschieben der Spannhülse in Richtung des am Ende des Schaftes sitzenden Werkzeuges werden die Greiforgane durch die Spannhülse radial nach innen gedrückt, so dass der Schraubenkopf von diesen im wesentlichen hakenförmig ausgebildeten Greiforganen hintergriffen und gegen das Werkzeug am Schaftende festsitzend gehalten wird. In ähnlicher Weise sind auch die Gegenstände der oben weiter genannten Druckschriften ausgestaltet.

Es hat sich nun gezeigt, dass die Handhabung solcher spezieller Schraubendreher insoweit kompliziert ist, als diese Schraubendreher zunächst mit den Werkzeugklingen ihres Werkzeuges auf den Schraubenkopf aufgesetzt werden müssen und anschließend die Spannhülse entlang des Schaftes verschoben werden muß, um den Schraubenkopf der Knochenschraube zu ergreifen. Da die hakenförmigen Greiforgane den Schraubenkopf hintergreifen, ist es mit diesem Werkzeug nicht möglich eine Knochenschraube vollständig einzuschrauben. Kurz vor erreichen der maximalen Einschraubtiefe muß die Spannhülse wieder zurückgezogen werden, um den Schraubenkopf freizugeben. Anschließend müssen auch die Greiforgane axial zurückgezogen werden, so dass diese beim weiteren Eindrehen der Knochenschraube nicht am Knochen anstehen und somit das weitere, vollständige Einschrauben der Knochenschraube nicht behindert wird. Gleichzeitig müssen die Greiforgane aus ihrer über das Werkzeug axial vorstehenden Position zurückgezogen werden, um das vollständige Einschrauben nicht zu behindern. Wird nun allerdings die Spannhülse zusammen mit den Greiforganen in eine unwirksame Stellung gebracht, so kann der Schraubendreher mit seinem klingenförmigen Werkzeug abrutschen, wodurch die Arbeitssicherheit vermindert wird.

Aus der DE 296 11 140 U1 ist ein System aus Schraubendreher und Knochenschraube bekannt, bei welchem das Werkzeug des Schraubendrehers einen zentralen Sechskant aufweist, welcher zwischen zwei radial nach außen stehenden Werkzeugklingen angeordnet ist. Dieser Sechskant steht in axialer Richtung minimal über die Werkzeugklingen hinaus und kann mit einem entsprechenden Innensechskant der Knochenschraube in Eingriff gebracht werden. Dabei ist der Sechskant des Werkzeuges konisch ausgebildet, so dass dieser bei entsprechendem Druck gegen die Knochenschraube in deren Innensechskant klemmend aufgenommen wird. Nachteilig an dieser Konstruktion ist, dass sich nach mehrfachem Einstecken des Sechskantes des Werkzeuges in einen Innensechskant der Knochenschraube am Sechskant des Werkzeuges ein Grat ausbildet, so dass dieses Werkzeug nach mehrfachem Gebrauch nicht mehr funktionsfähig ist, da es nicht mehr in den Innensechskant der Knochenschrauben tief genug einsteckbar ist sondern am Grat ansteht. Damit kann aber auch kein klemmender Halt mehr erzielt werden.

Beim Gegenstand der DE 93 10 668.8 werden Klemmelemente verwendet, welche als separate Bauteile am Werkzeug federelastisch gelagert sind. Zum einen ist hier eine Klemmkugel vorgesehen, welche in einer Bohrung des Werkzeuges eingesetzt ist. Als Werkzeug kann hier ein Innensechskant oder auch ein Außensechskant vorgesehen sein. Nachteilig ist hier, dass diese Konstruktion bei äußerst kleinen Knochenschrauben nicht einsetzbar ist, da die Klemmelemente stets einen äußerst großen axialen Abstand von der äußeren Stirnseite des Werkzeuges haben müssen. Dies hat zur Folge, dass die Knochenschrauben einen entsprechend langen Außensechskant oder Innensechskant aufweisen müssten, damit das Klemmelement überhaupt mit der Knochenschraube klemmend in Eingriff gelangen kann. Bei äußerst kleinen Knochenschrauben ist aber eine große axial Länge des Außensechskantes oder Innensechskantes nicht möglich, da diese Schrauben in ihren Abmessungen für den Einsatz in der Microchirurgie äußerst klein sein müssen.

In der DE 91 10 576 U1 ist ein Schraubendreher beschrieben, bei welchem innerhalb des Werkzeugschaftes ein Federstab vorgesehen ist, welcher mit den Klingenschlitzen der Knochenschraube durch seine radiale Federkraft klemmend in Eingriff gebracht werden soll. Auch diese Konstruktion ist in der Handhabung äußerst unsicher, da ein sicheres Klemmen nicht immer erreicht werden kann. Außerdem ist auch bei den genannten äußerst kleinen Knochenschrauben diese Konstruktion nicht realisierbar, da der Werkzeugschaft mit seinem äußerst kleinen Durchmesser eine Anordnung eines solchen Federstabes nicht zuläßt.

Schließlich ist aus der US 2,015,878 ein Schraubendreher bekannt, der die Merkmale des Oberbegriffs des Anspruchs 1 aufweist.

Demgemäß liegt der Erfindung die Aufgabe zu Grunde, einen Schraubendreher anzugeben, bei welchem die Handhabung einer Knochenschraube erheblich vereinfacht wird und welcher auch für äußerst kleine Abmessungen realisierbar ist.

Die Aufgabe wird erfindungsgemäß durch die Merkmale des Anspruchs 1 gelöst.

Durch diese Ausgestaltung bilden die Werkzeugklingen selbst die eigentlichen Klemmelemente, so dass das Werkzeug auch bei kleinen Abmessungen sicher realisierbar ist. Insbesondere können sowohl das Werkzeug als auch die zum System gehörige Knochenschraube mit ihren Kupplungselementen mit einer ausreichenden Stabilität versehen werden.

Weiter erübrigen sich damit im Gegensatz zum Eingangs beschriebenen Stand der Technik weitere Handgriffe am Schraubendreher, um die Knochenschraube über ihren Schraubenkopf einerseits festsitzend am Schraubendreher zu halten und andererseits um diese Verbindung wieder aufzuheben. Weiter vorteilhaft ist, dass mittels der besonderen erfindungsgemäßen Ausgestaltung zum vollständigen Einschrauben der Knochenschraube auch keine weiteren Aktionen der Bedienungsperson, wie beispielsweise ein Zurückziehen einer Spannhülse oder dergleichen, notwendig sind, um die Knochenschraube vollständig eindrehen zu können.

Für einen zentrierten, klemmenden Halt des Werkzeuges an der Knochenschraube ist vorgesehen, dass die Klingenschlitze der Knochenschraube in einer zentralen, zylindrischen Axialbohrung münden, und dass die Klemmabschnitte der Werkzeugklingen aus jeweils axial aus den Stirnflächen der Werkzeugklingen hervorstehenden, elastisch nachgiebigen Klemmelementen gebildet sind, deren Klemmflächen auf einem Außendurchmesser liegen, der dem Durchmesser der Axialbohrung zum Erzielen einer Klemmverbindung angepasst ist.

Durch diese erfindungsgemäße Ausgestaltung sowohl des Schraubenkopfes der Knochenschraube als auch des Werkzeuges des Schraubendrehers ist die Knochenschraube in einfachster Weise handhabbar. Dazu weist der Schraubenkopf zwischen seinen Klingenschlitzen eine zylindrische Axialbohrung auf, in welche die Klingenschlitze münden. Der Schraubendreher ist seinerseits mit entsprechenden Werkzeugklingen versehen, welche passend in die Klingenschlitze des Schraubenkopfes formschlüssig eingreifen. Jede der Werkzeugklingen weist jeweils ein axial über die Stirnfläche der jeweiligen Werkzeugklinge vorstehendes Klemmelement auf, mit welchen der Schraubendreher beim Ansetzen an den Schraubenkopf mit der zylindrischen Axialbohrung des Schraubenkopfes in Eingriff bringbar ist. Dabei sind diese elastischen Klemmelemente in ihrem Außendurchmesser derart ausgebildet, dass zwischen den Klemmelementen und der Axialbohrung eine Klemmverbindung erzielt wird.

Für die Handhabung bedeutet dies, dass der erfindungsgemäße Schraubendreher mit den Werkzeugklingen und den Klemmelementen seines Werkzeuges in einfacher Weise zentriert am Schraubenkopf ansetzbar ist, wobei beim Ansetzen gleichzeitig eine klemmende, den Schraubenkopf am Werkzeug festhaltende Verbindung entsteht. Insoweit ist eine Knochenschraube der erfindungsgemäßen Art mit dem Schraubendreher der erfindungsgemäßen Art in einfacher Weise aus einem Magazin entnehmbar und kann sodann in der gleichen einfachen Weise am Einsatzort in einen Knochen eingeschraubt werden. Die Klemmkräfte sind dabei derart ausgelegt, dass nach dem Einschrauben der Knochenschraube der Schraubendreher mit seinem Werkzeug und seinen Klemmelementen in einfachster Weise vom Schraubenkopf der Knochenschraube axial wieder abziehbar ist.

Es kann vorgesehen sein, dass die zwischen den einzelnen Klingenschlitzen liegenden inneren Begrenzungsflächen der durch die Klingenschlitze gebildeten Ringsektoren Teilflächen eines Zylinders bilden, dessen Durchmesser um den Faktor 0,05 bis 0,2 größer ist als der Durchmesser der Axialbohrung. Durch diese Ausgestaltung wird eine ausreichende Klemmkraft erreicht.

Die axialen Stirnflächen der Ringsektoren können Teilflächen eines Kegelmantels bilden, dessen Kegelwinkel zwischen 145° und 160° liegt. Durch diese Ausgestaltung ist auch ein "schräges" Ansetzen des Werkzeuges an der Knochenschraube möglich.

Weiter kann vorgesehen sein, dass sich die axialen Mittelebenen der Klingenschlitze in der Schraubenachse kreuzen, wobei die Breite der Klingenschlitze etwa einem Fünftel des Schraubenkopfdurchmessers entspricht. Durch diese Ausgestaltung ist die federelastische Nachgiebigkeit der Klemmabschnitte an die bestehenden Erfordernisse anpassbar, wobei durch die Breite der Klingenschlitze eine ausreichende Stabilität der Knochenschraube erhalten bleibt.

Es kann vorgesehen sein, dass der Durchmesser der zentralen Axialbohrung zumindest annähernd einem Drittel des Schraubenkopfdurchmessers entspricht, und dass die Tiefe der Axialbohrung dem zehnten bis achten Teil des Schraubenkopfdurchmessers entsprechen kann. Durch diese Ausgestaltung ist ein zentriertes Ansetzen des zugehörigen Werkzeuges mit seinen axial vorstehenden Klemmelementen sicher gewährleistet.

Die Werkzeugklingen des Schraubendrehers können jeweils durch gleichförmige, im Querschnitt dreieckige Ausschnitte eines rotationssymmetrischen Klingenschaftes gebildet sein und durch einen zentralen Schaftkern einstückig miteinander verbunden sein, dass wenigstens eine der Werkzeugklingen mit einem Trennschlitz versehen ist und ein radial außen liegendes Klemmelement bildet. Durch den Trennschlitz in der Werkzeugklinge wird eine äußerst einfache Herstellung des Klemmelementes auch bei äußerst kleinen Baugrößen erreicht, wobei ein klemmender Halt des Werkzeuges in den Klingenschlitzen einer Knochenschraube sichergestellt ist. Der Trennschlitz kann bei mehr als zwei Werkzeugklingen auch tangential zum Werkzeugkern verlaufend angeordnet sein, so dass zwei der Werkzeugklingen einstückig miteinander verbunden sind und die Drehkräfte auf die Knochenschraube übertragen, während die Klemmkräfte durch die "geschlitzte" Werkzeugklinge bewirkt werden, da diese zum zugehörigen Klingenschlitz der Knochenschraube in Umfangsrichtung leicht versetzt angeordnet ist.

Weiter können die Klemmelemente gemäß Anspruch 4 aus Sektoren eines zur Achse des Klingenschaftes konzentrischen Kreisringes bestehen, wobei die Sektoren des Kreisringes durch die Ausschnitte gebildet sind, welche die Werkzeugklingen bilden. Durch diese Ausgestaltung wird eine äußerst einfache Herstellung der Klemmelemente erreicht.

In gleicher Weise vorteilhaft ist auch die Ausgestaltung gemäß Anspruch 5, wonach vorgesehen sein kann, dass die Klemmelemente aus jeweils auf der Stirnfläche einer Werkzeugklinge liegenden Sektoren eines Kreisringes bestehen und durch radial verlaufende Nuten gebildet sind.

Die Klemmelemente können gemäß Anspruch 2 aus den Kreissektoren eines zentralen zylindrischen Zapfens bestehen, die durch wenigstens zwei sich kreuzende diametral verlaufende stirnseitig und radial offene Nuten gebildet sind, wobei sie in den Schaftkern eindringen und eine axiale Tiefe aufweisen können, die wenigstens doppelt so groß ist wie die axiale Höhe der Klemmelemente. Dabei kann vorgesehen sein, dass die Nuten jeweils in den Symmetrieebenen zweier sich diametral gegenüber liegenden Ausschnitte liegen. Durch diese Ausgestaltung erhalten die Klemmelemente eine zusätzlich elastische Nachgiebigkeit in radialer Richtung.

Hierbei kann der Schaftkern ein Durchmesser aufweisen, der mindestens gleich groß ist wie der Durchmesser des Zapfens, aus dem die Kreissektoren gebildet sind, wodurch Herstellung und Stabilität verbessert werden.

Weiter kann gemäß Anspruch 3 vorgesehen sein, dass weniger Kreissektoren vorhanden sind als Werkzeugklingen, wobei von sechs Werkzeugklingen zwei sich diametral gegenüberliegende Werkzeugklingen auf ihren Stirnflächen separate Kreissektoren aufweisen und jeweils die Stirnflächen zweier in Umfangsrichtung dazwischen liegender Werkzeugklingen gemeinsam einen Kreissektor tragen.

Gemäß Anspruch 6 ist vorgesehen, dass die Klemmelemente eine sich zur Ebene der Stirnflächen der Werkzeugklingen hin um 2° bis 6° konisch verjüngende Umfangsflächen und eine angeschrägte oder gerundete Randkante aufweisen. Durch diese Ausgestaltung ist die Klemmwirkung der Klemmelemente in der Axialbohrung der Knochenschraube verbessert.

Weiter kann vorgesehen sein, dass zumindest eine der Werkzeugklingen zumindest in ihrem axial äußeren, in die Klingenschlitze eingreifenden Endbereich einen vom Verlauf der Klingenschlitze derart abweichenden Verlauf aufweist, dass die Werkzeugklingen mit den Klingenschlitzen klemmend in Eingriff bringbar sind. Dies bedeutet, dass die Werkzeugklingen beispielsweise bei radial geradlinig verlaufenden Klingenschlitzen der Knochenschraube einen bogenförmigen Verlauf aufweisen können. Die Breite der Werkzeugklingen entspricht dabei etwa der Breite der Klingenschlitze der Knochenschraube. Beim Ansetzen der Werkzeugklingen an den Klingenschlitzen findet eine geringfügige federelastische Verformung der Werkzeugklingen statt, so dass die Werkzeugklingen klemmend in den Klingenschlitzen gehalten werden und somit die Knochenschraube in einfacher Weise am Schraubendreher bzw. dessen Werkzeug gehalten sind. Damit bildet die Werkzeugklinge auf Grund ihrer Formgebung selbst ein Klemmelement und ist dementsprechend formstabil und federelastisch ausgebildet.

Durch diese Ausgestaltung kann insbesondere bei kleinen Baugrößen auf axial vorstehende Klemmelemente an den Werkzeugklingen verzichtet werden sowie auch auf eine zentrale Axialbohrung in der Knochenschraube.

Weiter kann ein Winkelversatz einer der Werkzeugklingen in Umfangsrichtung vorgesehen sein, so dass diese Werkzeugklinge zum zugehörigen Klingenschlitz der Knochenschraube in Umfangsrichtung leicht versetzt angeordnet ist, während die weiter vorgesehenen Werkzeugklingen bezüglich ihrer Winkelausrichtung mit den jeweils zugeordneten Klingenschlitzen in Überdeckung sind.

Auch kann eine der Werkzeugklingen parallel zu einer Radialen verlaufend in Umfangsrichtung versetzt angeordnet sein, während die weiter vorgesehen Werkzeugklingen mit.den zugehörigen Klingenschlitzen in Überdeckung ausgerichtet sind. Auch kann eine der Werkzeugklingen beispielsweise im radial äußeren Bereich mit einer stirnseitig eingebrachten Nut versehen sein, so dass die Werkzeugklinge ein äußeres Klemmelement bildet. Sind beispielsweise vier Werkzeugklingen vorgesehen, so können zwei sich diametral gegenüberliegende Werkzeugklingen mit einer solchen Nut versehen sein, so dass eine symmetrische Klemmung und somit ein sicherer Halt des Werkzeuges an der Knochenschraube bewirkt wird.

Dabei kann die Formabweichung äußerst gering sein und im Bereich zwischen 0,025 mm und 0,25 mm liegen. Diese geringfügige Formabweichung ist einerseits für einen ausreichenden Klemmsitz ausreichend und andererseits werden die Werkzeugklingen beim Einsetzen in die Klingenschlitze nur geringfügig elastisch verformt, so dass die Werkzeugklingen keine bleibende Deformierung erleiden. Dabei ist das Maß der Formabweichung von der Gesamtgröße der Werkzeugklingen sowie der zu betätigenden Knochenschraube abhängig.

Weiter kann vorgesehen sein, dass das Werkzeug einen zentralen Schaftkern aufweist, welcher mit einer oder mehreren, die Werkzeugklingen zumindest in ihrem mit den Klingenschlitzen in Eingriff bringbaren Endbereichen trennenden, axialen Ausnehmungen versehen ist. Diese Ausgestaltung kann insbesondere bei Werkzeugen mit drei oder mehr Werkzeugklingen vorgesehen sein. Durch diese axiale Ausnehmung werden die federelastischen Eigenschaften der Werkzeugklingen gewährleistet.

Dabei kann vorgesehen sein, dass die Ausnehmung aus einer, zentralen stirnseitig in den Schaftkern eingebrachten Sacklochbohrung gebildet ist, die ausgehend von den axial äußeren Stirnflächen der Werkzeugklingen eine axiale Tiefe von 0,5 bis 2,5 mm aufweist. Durch diese Ausgestaltung.wird eine äußerst einfache Herstellbarkeit erreicht. Die Wahl der axialen Tiefe von etwa 0,5 bis 2,5 ist im wesentlichen von der Gesamtgröße der Werkzeugklingen, insbesondere deren radialer Länge und deren Breite abhängig. Bei größeren Abmessungen der Werkzeugklingen kann die axiale Tiefe der Ausnehmung einen größeren Wert und bei kleinen Abmessungen der Werkzeugklingen einen geringeren Wert annehmen. Durch die axiale Tiefe wird einerseits die Formstabilität, welche mit zunehmender Tiefe abnimmt und andererseits die Elastizität, welche mit zunehmender Tiefe zunimmt, der Werkzeugklingen beeinflußt.

Alternativ dazu können die Ausnehmungen auch aus radial verlaufenden Nuten gebildet sein, welche ausgehend von den axial äußeren Stirnflächen der Werkzeugklingen eine axiale Tiefe von 0,5 bis 2,5 mm aufweisen. Die Wirkungsweise dieser derart ausgebildeten Ausnehmungen ist identisch mit denen gemäß Anspruch 25, jedoch kann die Fertigung insbesondere bei gerader Anzahl von Werkzeugklingen vorteilhafter sein.

Zur Vereinfachung des Ansetzens der Werkzeugklingen an den Klingenschlitzen bzw. an der Knochenschraube kann vorgesehen sein, dass im Bereich des Schaftkerns ein axial über die Werkzeugklingen vorstehender Zentrierzapfen vorgesehen ist, dessen Durchmesser größer ist, als der Schaftkern und, dass der Zentrierzapfen in an der Knochenschraube angesetztem Zustand des Werkzeuges in eine zentrale Axialbohrung der Knochenschraube eingreift.

Gemäß einer weiteren Ausgestaltung werden "Winkelfehler" beim manuellen Eindrehen einer Knochenschraube ausgeglichen. Mit Winkelfehler ist hier eine nicht koaxiale Ausrichtung des Werkzeugschaftes zur Längsmittelachse der einzudrehenden Knochenschraube gemeint. Bei einer manuellen Betätigung des Schraubendrehers treten stets gewisse "Taumelbewegungen" des Schraubendrehers auf, welche durch die besondere Formgebung einerseits der Werkzeugklingen und andererseits der Klingenschlitze wirksam ausgeglichen werden. Dazu ist vorgesehen, dass die axial äußeren Stirnflächen der Werkzeugklingen ausgehend vom Schaftkern unter einem Kegelwinkel δ von 3° bis 10° zu einer quer zur Längsmittelachse des Schaftkerns verlaufenden Ebene schräg nach außen, zurückversetzt geradlinig oder bogenförmig verlaufen und, dass die Grundflächen der zugeordneten Klingenschlitze der Knochenschraube denselben Verlauf aufweisen.

Durch die erfindungsgemäße Ausgestaltung gemäß der oben angegebenen Schutzansprüche im Einzelnen und in Kombination wird ein Schraubendreher zur Verfügung gestellt, welcher in äußerst einfacher Weise sicher handhabbar ist, indem das Werkzeug des Schraubendrehers durch eine durch Klemmelemente des Werkzeuges bewirkte klemmende Steckverbindung mit der Knochenschraube verbindbar und wieder lösbar ist. Als Klemmelemente sind hier Teile der Werkzeugklingen selbst vorgesehen, die radial verlaufende Abschnitte der Werkzeugklinge sein können und/oder auch als eine Art geteilter Zentrierzapfen ausgebildet sein können, welcher an den Werkzeugklingen axial vorstehend angeformt ist.

Anhand der Zeichnung wird nachfolgend die Erfindung näher erläutert. Die nachfolgend angeführten Ausführungsformen, dienen lediglich beispielhaft zur Erläuterung der Erfindung. Es zeigt:
- Fig. 1: einen Schraubendreher in Seitenansicht mit einem Ausführungsbeispiel eines erfindungsgemäßen Werkzeuges;
- Fig. 2: einen vergrößerten Ausschnitt II des Werkzeuges des Schraubendrehers aus Fig. 1 ;
- Fig. 3: eine Stirnansicht III des Werkzeuges aus Fig. 2;
- Fig. 4: eine perspektivische Darstellung des Werkzeuges aus den Fig. 2 und 3;
- Fig. 5: eine erfindungsgemäße Knochenschraube, welche mit dem Werkzeug des Schraubendrehers aus Fig. 1 betätigbar ist in Seitenansicht;
- Fig. 6: eine Draufsicht des Schraubenkopfes der Knochenschraube aus Fig. 5;
- Fig. 7: eine perspektivische Ansicht des Schraubenkopfes aus Fig. 6;
- Fig. 8: die perspektivische Darstellung des Werkzeuges aus Fig. 4 zusammen mit einem Teilschnitt des Schraubenkopfes aus Fig. 7 in Rückansicht;
- Fig. 9: den Schraubenkopf aus Fig. 8 im Eingriff mit dem Werkzeug aus Fig. 8;
- Fig. 10: ein zweites Ausführungsbeispiel eines Werkzeuges mit drei Werkzeugklingen;
- Fig. 11: ein weiteres Ausführungsbeispiel eines Werkzeuges mit sechs Werkzeugklingen;
- Fig. 12: ein Ausführungsbeispiel eines Werkzeuges mit ebenfalls sechs Werkzeugklingen und insgesamt vier Klemmelementen;
- Fig. 13: eine Draufsicht XIII des Werkzeuges aus Fig. 12;
- Fig. 14: einen Teilschnitt durch ein Klemmelement des Werkzeuges aus Fig. 10;
- Fig. 14a: das Klemmelement aus Fig. 14 im Eingriff mit dem Schraubenkopf aus Fig. 15;
- Fig. 15: eine perspektivische Darstellung eines Schraubenkopfes mit drei Radialschlitzen, welcher mit dem Werkzeug aus Fig. 10 in Eingriff bringbar ist;
- Fig. 16: eine perspektivische Darstellung eines Schraubenkopfes, welcher mit insgesamt sechs Radialschlitzen versehen ist, welcher wahlweise mit dem Werkzeug aus Fig. 11 oder dem Werkzeug aus Fig. 12 in Eingriff bringbar ist;

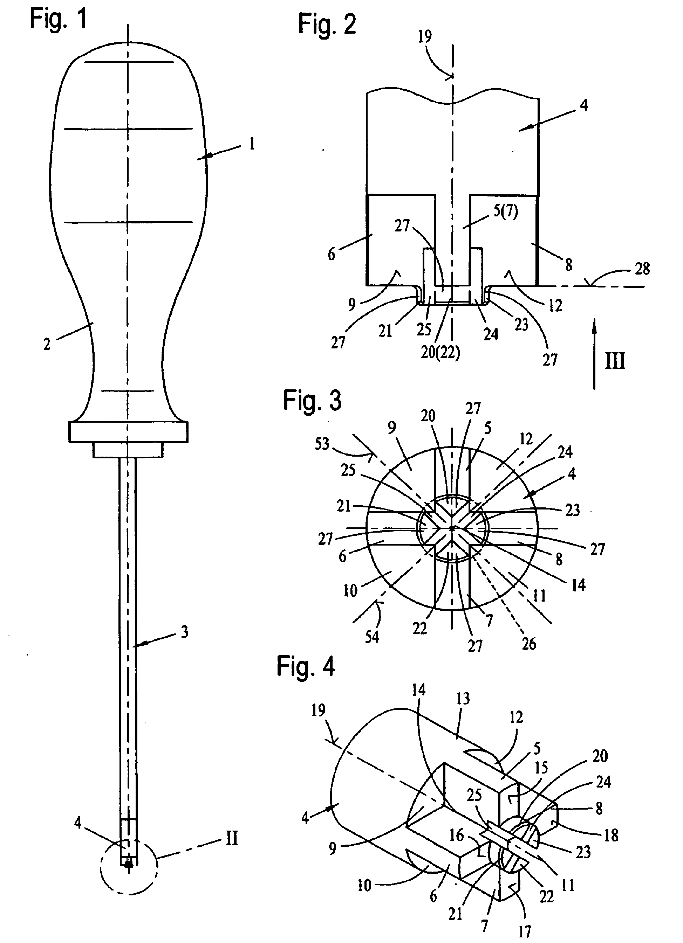

Fig. 1 zeigt eine Seitenansicht eines Schraubendrehers 1, welcher in bekannter Weise zur Betätigung ein Griffteil 2 aufweist. Dieses Griffteil 2 steht mit einem Werkzeugschaft 3 in drehfester Verbindung, welcher an seinem, dem Griffteil 2 gegenüberliegenden Ende ein Werkzeug 4 aufweist, das zur drehenden Betätigung einer Knochenschraube dient. Dabei kann vorgesehen sein, dass der Werkzeugschaft 3 gegen weitere Werkzeugschäfte mit unterschiedlichen Werkzeugen austauschbar am Griffteil 2 befestigt ist.

Aus Fig. 2 ist eine vergrößerte Darstellung II dieses Werkzeuges 4 aus Fig. 1 ersichtlich, wobei in Zusammenhang mit Fig. 3 erkennbar ist, dass dieses Werkzeug 4 insgesamt vier radial verlaufende Werkzeugklingen 5, 6, 7 und 8 aufweist. Diese vier Werkzeugklingen 5 bis 8 werden durch im Querschnitt im wesentlichen dreieckförmige Ausschnitte 9, 10, 11 und 12 gebildet.

Wie aus den Fig. 3 und 4 ersichtlich ist, bildet das Werkzeug 4 einen rotationssymmetrischen, zylindrischen Klingenschaft 13, in welchem die Ausschnitte 9 bis 12 angeordnet sind. Des weiteren ist aus den Fig. 3 und 4 erkennbar, dass diese Werkzeugklingen 5 bis 8 durch einen zentralen Schaftkern 14 einstückig miteinander verbunden sind. Die Werkzeugklingen 5 bis 8 sind jeweils stegartig ausgebildet und enden in axialer Richtung mit ihren jeweiligen, ebenen Stirnflächen 15, 16, 17 und 18 in einer gemeinsamen Ebene 28, welche rechtwinklig zur Längsmittelachse 19 des Werkzeuges 4 verläuft, wie dies insbesondere aus Fig. 2 ersichtlich ist.

Auf jeder der Stirnflächen 15 bis 18 jeder Werkzeugklinge 5 bis 8 ist jeweils ein Klemmelement 20, 21, 22 und 23 vorgesehen, welche die jeweiligen Stirnflächen 15 bis 18 in axialer Richtung nach außen überragen. Diese Klemmelemente 20 bis 23 werden beim vorliegenden Ausführungsbeispiel durch zwei sich rechtwinklig kreuzende, diametral verlaufende, stirnseitig und radial offene Nuten 24 und 25 gebildet. D.h., dass die Klemmelemente 20 bis 23 beim vorliegenden Ausführungsbeispiel des Werkzeuges 4 aus vier Zylinderabschnitten 27 eines zentralen, zylindrischen Zapfens 26 bestehen, welcher in Fig. 3 gestrichelt dargestellt ist. Die axiale Tiefe der beiden Nuten 24 und 25 ist größer ausgebildet als die axiale Höhe der vier Klemmelemente 20 bis 23, so dass die beiden Nuten 24, 25 in axialer Richtung bis in den Schaftkern 14 des Klingenschaftes 13 hineinreichen. Weiter liegen die Nuten 24, 25 jeweils in den Symmetrieebenen 53 bzw. 54 zweier sich diametral gegenüber liegenden Ausschnitte 9, 11 bzw. 10, 12, wie aus Fig. 3 erkennbar ist. Der die Klemmelemente 20 bis 23 bildende zentrale Zapfen 26 weist beim vorliegenden Ausführungsbeispiel einen Durchmesser auf, welcher größer ist als der Durchmesser des Schaftkernes 14.

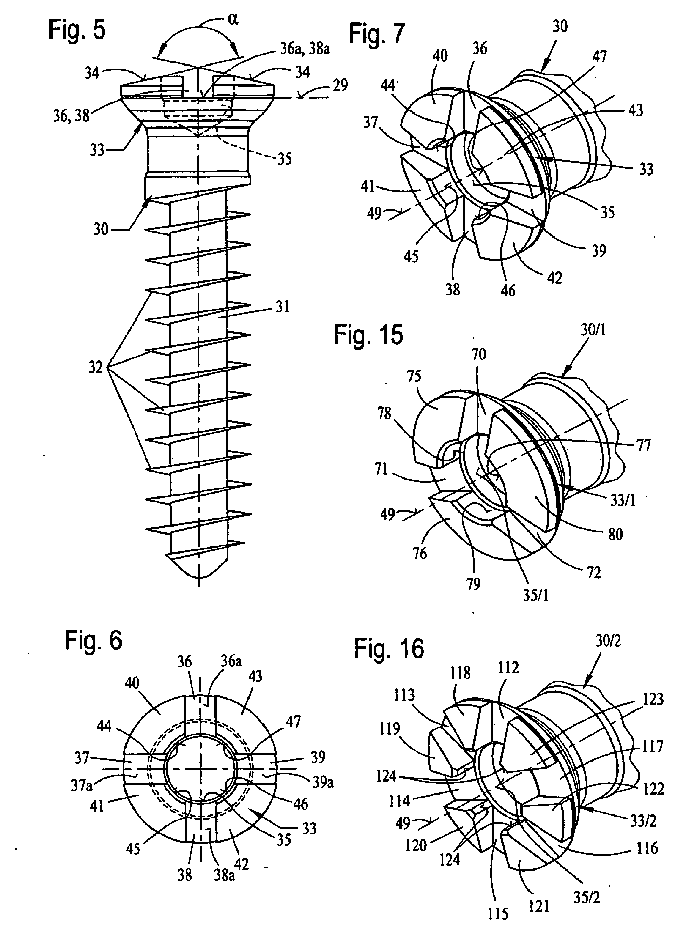

Fig. 5 zeigt beispielhaft eine mittels des Werkzeuges 4 betätigbare Knochenschraube 30, welche mit einem Gewindeschaft 31 mit Außengewinde 32 versehen ist, das einen Durchmesser von etwa 2 mm aufweist. Dieses Außengewinde 32 ist beim vorliegenden Ausführungsbeispiel als selbstschneidendes Gewinde ausgebildet.

Am Gewindeschaft 31 ist in Fig. 5 am oberen Ende ein Schraubenkopf 33 angeformt, welcher radial erweitert ausgebildet ist und einen Durchmesser von etwa 3 mm aufweist. Die obere Stirnfläche 34 des Schraubenkopfes 33 bildet eine Art Kegelmantel, dessen Kegelwinkel α zwischen 145° und 160° liegt. Aus den Fig. 5 und 6 ist weiter erkennbar, dass der Schraubenkopf 33 eine zentrale, zylindrische Axialbohrung 35 aufweist, welche als Sacklochbohrung ausgebildet ist. Der Schraubenkopf 33 ist beim vorliegenden Ausführungsbeispiel mit insgesamt vier radial verlaufenden Klingenschlitzen 36, 37, 38 und 39 versehen, die auf Grund der "Kegelform" der äußeren Stirnfläche 34 des Schraubenkopfes 33 eine radial von außen nach innen, entsprechend des vorgesehenen Kegelwinkels α ansteigende Tiefe aufweisen.

Diese Klingenschlitze 36 bis 39 münden, wie dies insbesondere aus Fig. 7 ersichtlich ist, in die Axialbohrung 35. Durch die Klingenschlitze 36 bis 39 werden, beim vorliegenden Ausführungsbeispiel insgesamt 4 Ringsektoren 40, 41, 42 und 43 gebildet. Die von diesen Ringsektoren zur Axialbohrung 35 hin gebildeten Teilflächen 44, 45, 46 und 47 sind radial um einen Faktor von 0,05 bis 0,02 in ihrem Durchmesser größer ausgebildet als der Durchmesser der Axialbohrung 35. Die Klingenschlitze 36 bis 39 weisen jeweils eine U-förmige Querschnittsform mit einer quer zur Längsmittelachse 49 der Knochenschraube 30 verlaufenden Grundfläche 36a, 37a, 38a und 39a (Fig. 6) auf, welche in einer gemeinsamen Planebene 29 liegen (Fig. 5). Die axiale Tiefe der Axialbohrung 35 kann dabei ausgehend von der gemeinsamen Planebene 29 der Grundflächen 36a bis 39a wenigstens gleich groß oder größer sein wie die axial Maximaltiefe der Klingenschlitze 36 bis 39 des Schraubenkopfes 33. Weiter kann auch vorgesehen sein, dass die Tiefe der Axialbohrung 35 wenigstens dem zehnten bis achten Teil des Durchmessers des Schraubenkopfes 33 entspricht.

Durch diese Ausgestaltung des Schraubenkopfes 33 mit seinen Klingenschlitzen 36 bis 39 und seiner Axialbohrung 35 sowie den radial erweiterten Teilflächen 44 bis 47 wird ein einfaches Ansetzen des Werkzeuges 4 mit seinen Klemmelementen 20 bis 23 in der Axialbohrung 35 ermöglicht.

Es ist leicht vorstellbar, dass beim Ansetzen des Werkzeuges 4 am Schraubenkopf 5 dieses zum einen mit seinen Werkzeugklingen 5, 6, 7 und 8 mit den Klingenschlitzen 36, 37, 38 und 39 des Schraubenkopfes 33 formschlüssig in Eingriff bringbar ist. Des weiteren gelangen beim Ansetzen des Werkzeuges 4 am Schraubenkopf 33 die Klemmelemente 20 bis 23 mit der Axialbohrung 35 klemmend in Eingriff.

Dabei ist der Außendurchmesser der Klemmelemente 20 bis 23 dem Innendurchmesser der Axialbohrung 35 derart angepaßt, dass ein klemmender Sitz der Klemmelemente 20 bis 23 in der Axialbohrung 35 erreicht wird. Dies bedeutet, dass nach dem Ansetzen des Werkzeuges 4 am Schraubenkopf 33 die Knochenschraube 30 über die Axialbohrung 35 ihres Schraubenkopfes 33 klemmend und somit unverlierbar am Werkzeug 4 gehalten wird. Da die Abmessungen, sowohl der Axialbohrung 35 als auch der Klemmelemente 20 bis 23, in ihrer axialen Länge derart aufeinander abgestimmt sind, dass die Werkzeugklingen 5 bis 8 sicher formschlüssig mit den Klingenschlitzen 36 bis 39 in Eingriff gelangen, ist somit ein sicheres Betätigen der Knochenschraube 30 gewährleistet.

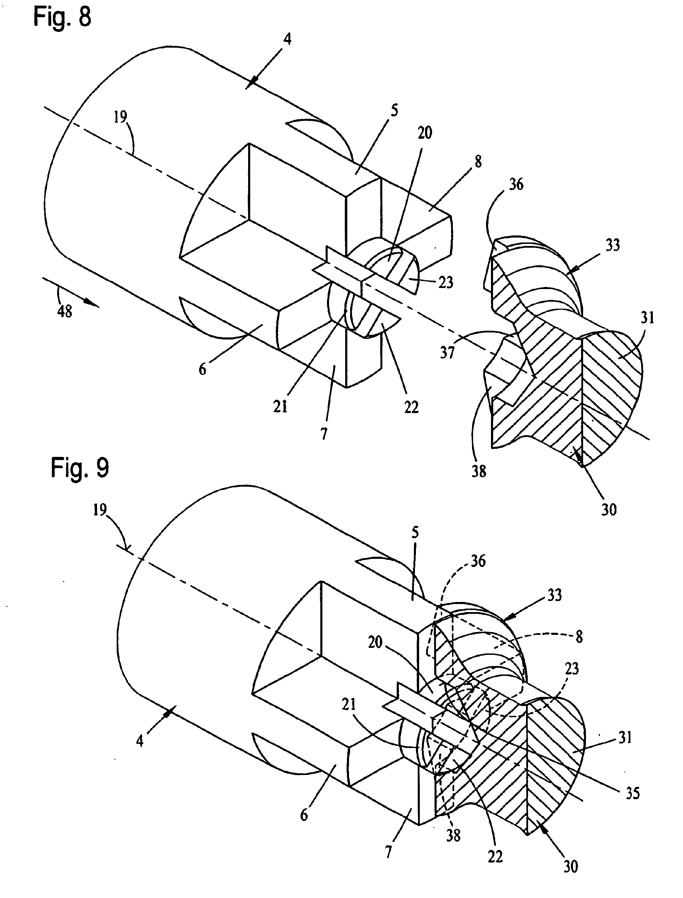

Fig. 8 zeigt eine vergrößerte Darstellung des Werkzeuges 4, wobei hier die Werkzeugklingen 5 bis 8 auf den im Teilschnitt dargestellten, vom Schraubenschaft 31 her betrachteten Schraubenkopf 33 ausgerichtet sind. Bei dieser Ausrichtung ist die Werkzeugklinge 5 auf den Klingenschlitz 36, die Werkzeugklinge 8 auf den Klingenschlitz 39, die Werkzeugklinge 7 auf den Klingenschlitz 38 und die Werkzeugklinge 6 auf den in Fig. 8 nicht sichtbaren Klingenschlitz 39 ausgerichtet.

Wird nun das Werkzeug 4 durch Bewegung in Richtung des Pfeiles 48 in axialer Richtung mit dem Schraubenkopf 33 in Kontakt gebracht, gelangen die Werkzeugklingen 5 bis 8 formschlüssig in die entsprechenden Klingenschlitze 36 bis 39 des Schraubenkopfes 33, wie dies aus Fig. 9 ersichtlich ist. Gleichzeitig gelangen die vier Klemmelemente 20 bis 23 in die Axialbohrung 35, in welcher diese Klemmelemente 20 bis 23 aufgrund ihres geringen, radialen Übermaßes klemmend gehalten sind. Es ist leicht vorstellbar, dass aufgrund dieser Kombination der Klemmelemente 20 bis 23 die Schraube 30 mit ihrem Schraubenkopf 33 klemmend am Werkzeug 4 gehalten wird. Damit ist die Knochenschraube 30 einerseits in einfacher Weise aus einem entsprechenden Schraubenmagazin entnehmbar und andererseits auch in sicherer Weise in einen Knochen einschraubbar.

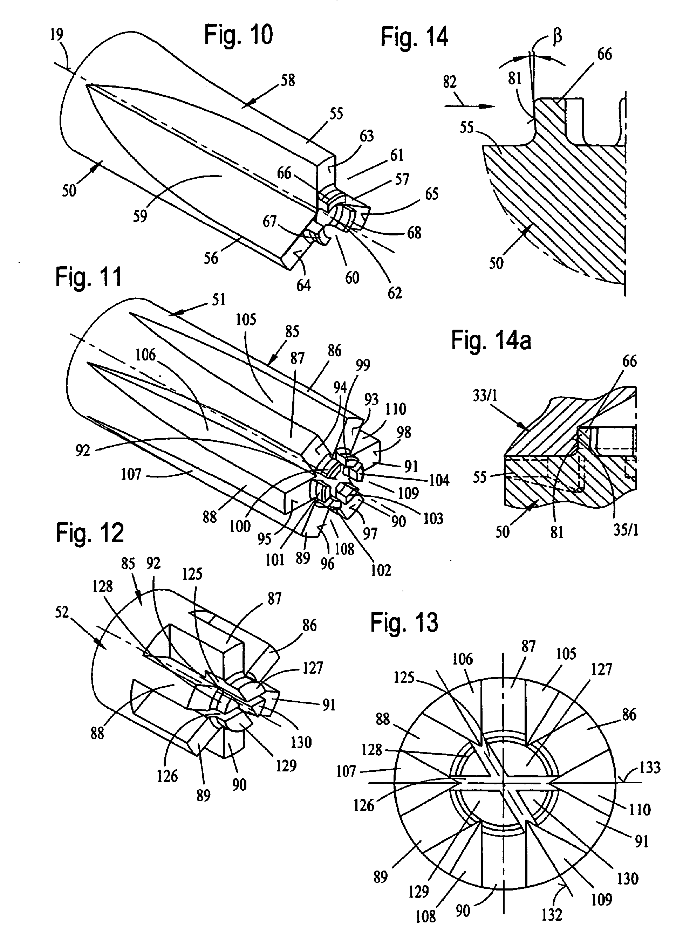

In den Fig. 10 bis 13 sind weitere Ausführungsbeispiele von Werkzeugen 50, 51 und 52 dargestellt.

Fig. 10 zeigt hierbei ein Werkzeug 50, das insgesamt drei radial verlaufende Werkzeugklingen 55, 56 und 57 aufweist. Das Werkzeug 50 wird ebenfalls durch einen zylindrischen Klingenschaft 58 gebildet, welcher mit drei Ausschnitten 59, 60 und 61 zur Bildung der drei Werkzeugklingen 55, 56 und 57 versehen ist. Auch die drei Werkzeugklingen 55, 56 und 57 sind über einen Schaftkern 62 einstückig miteinander verbunden.

Radial außerhalb des Schaftkernes 62 sind die drei Stirnflächen 63, 64 und 65 der drei Werkzeugklingen 55, 56 und 57 jeweils mit einem axial vorstehenden Ringsektor 66, 67 und 68 versehen, welche als Sektoren eines zur Längsmittelachse 19 des Klingenschaftes 58 konzentrischen Kreisringes (in der Zeichnung nicht explizit dargestellt) ausgebildet sind.

Dabei werden die Ringsektoren 66, 67 und 68 ebenfalls durch die drei Ausschnitte 59, 60 und 61 des Klingenschaftes 58 gebildet. Die Ringsektoren 66, 67 und 68 stellen hier die Klemmelemente des Werkzeuges 50 dar, welches mit einem entsprechend ausgestalteten Schraubenkopf 30/1, wie dieser in Fig. 15 dargestellt ist, in Eingriff bringbar ist. Dabei bilden diese Ringsektoren 66, 67 und 68 gleichzeitig auch eine Art Zentrierzapfen.

Hierzu weist der Schraubenkopf 30/1 ebenfalls eine zentrale Axialbohrung 35/1 auf. Des weiteren ist auch dieser Schraubenkopf 33/1 beim vorliegenden Ausführungsbeispiel mit insgesamt drei radial verlaufenden Klingenschlitzen 70, 71 und 72 versehen, welche in die Axialbohrung 35/1 münden. Durch die Klingenschlitze 70, 71 und 72 werden stirnseitig am Schraubenkopf 30/1 ebenfalls Ringsektoren 75, 76 und 77 gebildet, die zur Axialbohrung 35/1 hin durch bogenförmig verlaufende Teilflächen 78, 79 und 80 begrenzt sind. Der Innendurchmesser der auf dem gleichen Durchmesser liegenden Teilflächen 78, 79 und 80 ist hier ebenfalls um einen Faktor von 0,05 bis 0,2 größer ausgebildet als der Durchmesser der Axialbohrung 35/1.

Somit entspricht diese Ausgestaltung mit Ausnahme, dass hier lediglich drei Klingenschlitze 70 bis 72 vorgesehen sind, identisch der Ausgestaltung des Schraubenkopfes der Schraube 30 aus Fig. 5. Die Abmessungen, insbesondere der Außendurchmesser der Ringsektoren 66, 67 und 68 im Sinne der Klemmelemente 20 bis 23, sind dabei derart ausgebildet, dass diese Klemmelemente 66, 67 und 68 klemmend mit der Axialbohrung 35/1 des Schraubenkopfes 33/1 in Eingriff bringbar sind.

Gleichzeitig gelangen die drei Werkzeugklingen 55, 56 und 57 mit den Klingenschlitzen 70, 71 und 72 formschlüssig in Eingriff, so dass die Schraube 30/1 einerseits klemmend am Werkzeug 50 gehalten ist und andererseits sicher betätigbar ist.

Auch die vorderen Stirnflächen 63, 64 und 65 der Werkzeugklingen 55, 56 und 57 liegen in einer gemeinsamen Ebene(in der Zeichnung nicht explizit dargestellt), welche rechtwinklig zur Längsmittelachse 19 des Klingenschaftes 58 verläuft.

Fig. 14 zeigt einen Längsschnitt durch das Klemmelement 66, an welches sich radial nach außen die Werkzeugklinge 55 anschließt. Aus Fig. 14 ist ersichtlich, dass die äußere Mantelfläche 81 dieses Klemmelementes 66, ausgehend von der

Werkzeugklinge 55, unter einem Winkel β zum axial äußeren Ende hin von etwa 4° radial erweitert verläuft. Aufgrund des äußerst geringen Querschnittes des Klemmelementes 66 ist dieses in radialer Richtung leicht nachgiebig, so dass das Klemmelement 66 beim Einschieben in die Axialbohrung 35/1 geringfügig in Richtung des Pfeiles 82 gedrückt wird und somit ein klemmender Halt in der Axialbohrung 35/1 erreicht wird, wie dies in Fig. 14a dargestellt ist. Diese Ausgestaltung des Klemmelementes 66 gilt auch für die Klemmelemente 67 und 68.

Beim Ausführungsbeispiel des Werkzeuges 51 ist der Klingenschaft 85 mit insgesamt sechs Werkzeugklingen 86, 87, 88, 89, 90 und 91 versehen, welche ebenfalls ausgehend von einem zentralen Schaftkern 92 radial verlaufen. Diese Werkzeugklingen 86 bis 91 weisen auf ihrer jeweiligen Stirnseite 93, 94, 95, 96, 97, und 98 ebenfalls Klemmelemente 99, 100, 101, 102, 103 und 104 auf, welche, abgesehen von ihrer Umfangslänge, identisch ausgebildet sind, wie die als Ringsektoren ausgestalteten Klemmelemente 66 bis 68 der Werkzeugklingen 63 bis 65 aus Fig. 10.

Dies bedeutet, dass auch beim Klingenschaft 85 entsprechende Ausschnitte 105, 106, 107, 108, 109 und 110 vorgesehen sind, durch welche einerseits die Werkzeugklingen 86 bis 91 und andererseits die ebenfalls als Ringsektoren ausgestalteten Klemmelemente 99 bis 104 gebildet werden.

Das Werkzeug 51 ist mit seinen Werkzeugklingen 86 bis 91 mit entsprechenden Klingenschlitzen 112, 113, 114, 115, 116 und 117 eines entsprechend ausgestalteten Schraubenkopfes 33/2 einer Knochenschraube 30/2 formschlüssig in Eingriff bringbar. Ein solcher Schraubenkopf 33/2 ist beispielhaft in Fig. 16 dargestellt.

Durch diese Klingenschlitze 112 bis 117 werden am Schraubenkopf 33/2 ebenfalls Ringsektoren 118, 119, 120, 121, 122 und 123 gebildet. Die Klingenschlitze 112 bis 117 münden ebenfalls in eine Axialbohrung 35/2, mit welcher die Klemmelemente 99 bis 104 klemmend in Eingriff bringbar sind. Die inneren Teilflächen 124 der Ringsektoren 118 bis 123 liegen dabei ebenfalls auf einem um den Faktor 0,05 bis 0,2 größeren Durchmesser als der Durchmesser der Axialbohrung 35/2.

Es ist leicht vorstellbar, dass das Werkzeug 51 mit seinen Werkzeugklingen 86 bis 91 formschlüssig mit den Klingenschlitzen 112 bis 117 des Schraubenkopfes 33/2 in Eingriff bringbar ist, wie dies zum Ausführungsbeispiel des Werkzeuges 4 zusammen mit dem Schraubenkopf 33 in den Fig. 8 und 9 beschrieben wurde.

Gleichzeitig gelangen die als Ringsektoren ausgebildeten Klemmelemente 99 bis 104 klemmend mit der Axialbohrung 35/2 in Eingriff, so dass die Schraube 30/2 über ihren Schraubenkopf 33/2 am Werkzeug 51 gehalten wird.

Fig. 12 zeigt noch eine alternative Ausgestaltung eines Werkzeuges, welches ähnlich ausgebildet ist, wie das Werkzeug 51 aus Fig. 11.

Beim Werkzeug 52 sind ebenfalls sechs radial verlaufende Werkzeugklingen 86 bis 91 vorgesehen. Diese Werkzeugklingen 86 bis 91 werden auch hier durch sechs Ausschnitte 105 bis 110 gebildet, welche entsprechend der Ausschnitte 9, 10, 11 und 12 des Ausführungsbeispiels aus den Fig. 2 bis 4 ausgestaltet sind. Als Klemmelemente sind hier jedoch nicht Ringsektoren gemäß dem Ausführungsbeispiel der Fig. 11 vorgesehen, sondern durch zwei diametral verlaufende Nuten 125 und 126 gebildete Klemmelemente 127, 128, 129 und 130. Durch die Anordnung der Nuten 125 und 126 sind die beiden Klemmelemente 127 und 129 gemeinsamer Bestandteil der beiden Werkzeugklingen 86 und 87 bzw. 89 und 90. Die beiden in ihrer Umfangserstreckung kleiner ausgebildeten Klemmelemente 128 und 130 sind jeweils den Werkzeugklingen 88 und 91 zugeordnet.

Durch diese Ausgestaltung werden insbesondere aufgrund der größeren Umfangslänge der beiden Klemmelemente 127 und 129 stabilere Klemmelemente erreicht, so dass eine unzulässige Deformierung dieser Klemmelemente beim Ansetzen an den Schraubenkopf 33/2 aus Fig. 16 sicher verhindert wird. Durch die Verbindung der Werkzeugklingen 86, 87 und 89, 90 untereinander im Bereich des Schaftkernes 92 erhalten diese Werkzeugklingen 86, 87, 89, 90 eine höhere Steifigkeit, so dass diese auch bei größeren Drehkräften beim Einschrauben einer Knochenschraube nicht deformiert werden können.

Aus Fig. 13, welche eine Stirnansicht des Werkzeuges 52 darstellt, ist die genaue Anordnung sowohl der Werkzeugklingen 86 bis 91 als auch der Klemmelemente 127 bis 130 näher ersichtlich. Es ist erkennbar, dass die Nuten 125, 126 jeweils in den Symmetrieebenen 132 bzw. 133 zweier sich diametral gegenüber liegender Ausschnitte 106, 109 bzw. 107, 110 liegen, wie dies zum Ausführungsbeispiel der Fig. 2 bis 4 näher ausgeführt wurde. Dementsprechend erstrecken sich die Nuten 125 und 126 in axialer Richtung auch bis in den Schaftkern 92 des Klingenschaftes 85 hinein, wie dies aus Fig. 12 erkennbar ist.

Hier kann auch vorgesehen sein, dass die Werkzeugklingen 86 bis 91 ohne die Klemmelemente 127 bis 130 ausgebildet sind. In diesem Fall sind die beiden "einzelnen" Werkzeugklingen 88 und 91 in Umfangsrichtung leicht versetzt anzuordnen, so dass diese beiden Werkzeugklingen 88 und 91 selbst die Klemmelemente bilden, sofern die Klingenschlitze 114 und 117 der zugehörigen Knochenschraube 30/2 aus Fig. 16 nicht in Umfangsrichtung versetzt angeordnet sind.

An dieser Stelle sei nochmals bemerkt, dass die zu den einzelnen Ausführungsbeispielen beschriebenen speziellen Ausführungsformen sowohl allein als auch in beliebiger Kombination miteinander realisierbar sind. Allen Merkmalen und Merkmalskombinationen ist gemeinsam, dass das Werkzeug sicher klemmend und wieder lösbar an einer Knochenschraube gehalten wird und keinerlei weitere Vorrichtungen notwendig sind, um einen sicheren Halt des Werkzeuges am Schraubenkopf (oder umgekehrt) zu bewirken. Insbesondere sind an den Werkzeugklingen Klemmelemente vorgesehen, die auch durch die Werkzeugklingen selbst gebildet sein können.

## Patentansprüche

1. Schraubendreher (1) für eine Knochenschraube (30, 30/1, 30/2), der ein Werkzeug aufweist, das mit wenigstens drei sich radial und axial erstreckenden Werkzeugklingen (5, 6, 7, 8, 55, 56, 57, 86, 87, 88, 89, 90, 91) versehen ist, welche mit nutenartigen Klingenschlitzen (36, 37, 38, 39, 70, 71, 72, 112, 113, 114, 115, 116, 117) der Knochenschraube, die in einer zentralen, zylindrischen Axialbohrung (35, 35/1, 35/2) der Knochenschraube (30, 30/1, 30/2) münden, formschlüssig in Eingriff bringbar sind, wobei die Werkzeugklingen (5, 6, 7, 8, 55, 56, 57, 86, 87, 88, 89, 90, 91) wenigstens einen federelastischen Klemmabschnitt (20, 21, 22, 23, 66, 67, 68, 99, 104, 127, 128, 129, 130) bilden, mit welchem das Werkzeug (4, 50, 51, 52) im Bereich der Axialbohrung (35, 35/1) klemmend mit der Knochenschraube (30, 30/1, 30/2) in Eingriff bringbar ist,
**dadurch gekennzeichnet,**
**dass** die Klemmabschnitte der Werkzeugklingen (5, 6, 7, 8, 55, 56, 57, 86, 87, 88, 89, 90, 91) aus jeweils axial aus den Stirnflächen (15, 16, 17, 18, 63, 64, 65, 93, 94, 95, 96, 97, 98) der Werkzeugklingen (5, 6, 7, 8, 55, 56, 57, 86, 87, 88, 89, 90, 91) hervorstehenden, elastisch nachgiebigen Klemmelementen (20, 21, 22, 23, 66, 67, 68, 99, 100, 101, 102, 103, 104, 127, 128, 129, 130) gebildet sind, deren Klemmflächen (27, 81) auf einem Außendurchmesser liegen, der dem Durchmesser der Axialbohrung (35, 35/1, 35/2) des Schraubenkopfes der Knochenschraube (30, 30/1, 30/2) zum Erzielen einer Klemmverbindung angepasst ist.

2. Schraubendreher nach Anspruch 1, **dadurch gekennzeichnet, dass** die Klemmelemente (20, 21, 22, 23) aus den Kreissektoren (27) eines zentralen zylindrischen Zapfens (26) bestehen, die durch wenigstens zwei sich kreuzende, diametral verlaufende, stirnseitig und radial offene Nuten (24, 25, 125, 126) gebildet sind und dass die Nuten (24, 25, 125, 126) in den Schaftkern (14) eindringen und eine axiale Tiefe aufweisen, die wenigstens doppelt so groß ist wie die axiale Höhe der Klemmelemente (5, 6, 7, 8, 127, 128, 129, 130), und dass die Nuten (24, 25) jeweils in den Symmetrieebenen (53 bzw. 54) zweier sich diametral gegenüber liegenden Ausschnitte (9, 11 bzw. 10, 12) liegen.

3. Schraubendreher nach Anspruch 2, **dadurch gekennzeichnet, dass** weniger Kreissektoren (127, 128, 129, 130) vorhanden sind als Werkzeugklingen (86, 87, 88, 89, 90, 91), wobei von sechs Werkzeugklingen (86, 87, 88, 89, 90, 91) zwei sich diametral gegenüber liegende Werkzeugklingen (88, 91) auf ihren Stirnflächen separate Kreissektoren (128, 130) aufweisen und jeweils die Stirnflächen zweier in Umfangsrichtung dazwischen liegender Werkzeugklingen (86, 87 und 89, 90) gemeinsam einen Kreissektor (127, 129) tragen.

4. Schraubendreher nach Anspruch 1, **dadurch gekennzeichnet, dass** die Klemmelemente (66, 67, 68, 99, 100, 101, 102, 103, 104) aus axial vorstehenden Sektoren ((20, 21, 22, 23) eines zur Achse (19) des Klingenschaftes (58, 85) konzentrischen Kreisringes bestehen und dass die Sektoren (66, 67, 68, 99, 100, 101, 102, 103, 104) des Kreisringes durch die Ausschnitte (59, 60, 61, 105, 106, 107, 108, 109, 110) gebildet sind, welche die Werkzeugklingen (55, 56, 57, 86, 87, 88, 89, 90, 91) bilden. (Fig. 11)

5. Schraubendreher nach Anspruch 1, **dadurch gekennzeichnet, dass** die Klemmelemente aus jeweils auf der Stirnfläche (93, 94, 95, 96, 97, 98) einer Werkzeugklinge (86, 87, 88, 89, 90, 91) liegenden Sektoren (99, 100, 101, 102, 103, 104) eines Kreisringes bestehen und durch radial verlaufende Nuten gebildet sind.

6. Schraubendreher nach einem der Ansprüche 1 bis 5, **dadurch gekennzeichnet, dass** die Klemmelemente (66) eine sich zur Ebene der Stirnflächen (63) der Werkzeugklingen (55) hin unter einem Winkel (β) von 2° bis 6° konisch verjüngende Umfangsfläche (81) und eine angeschrägte oder gerundete Randkante aufweisen.

7. Schraubendreher nach einem der Ansprüche 2 oder 3, **dadurch gekennzeichnet, dass** die radial verlaufenden Nuten (24, 25, 125, 126) ausgehend von den axial äußeren Stirnflächen der Werkzeugklingen (5, 6, 7, 8, 86, 87, 88, 89, 90, 91) eine axiale Tiefe von 0,5 bis 2,5 mm aufweisen.

## Claims

1. A screwdriver (1) for a bone screw (30, 30/1, 30/2), which comprises a tool provided with at least three radially and axially extending tool blades (5, 6, 7, 8, 55, 56, 57, 86, 87, 88, 89, 90, 91), which with groove-like blade slots (36, 37, 38, 39, 70, 71, 72, 112, 113, 114, 115, 116, 117) of the bone screw, which open into a central cylindrical axial bore (35, 35/1, 35/2) of the bone screw (30, 30/1, 30/2), can be brought into engagement in a form-closed manner, wherein the tool blades (5, 6, 7, 8, 55, 56, 57, 86, 87, 88, 89, 90, 91) form at least one spring-resilient clamping section (20, 21, 22, 23, 66, 67, 68, 99, 104, 127, 128, 129, 130), with which the tool (4, 50, 51, 52) can be brought into engagement in the region of the axial bore (35, 35/1) in a clamping manner with the bone screw (30, 30/1, 30/2),
**characterised in that**
the clamping sections of the tool blades (5, 6, 7, 8, 55, 56, 57, 86, 87, 88, 89, 90, 91) are formed respectively from elastically flexible clamping elements (20, 21, 22, 23, 66, 67, 68, 99, 100, 101, 102, 103, 104, 127, 128, 129, 130) projecting axially from the front faces (15, 16, 17, 18, 63, 64, 65, 93, 94, 95, 96, 97, 98) of the tool blades (5, 6, 7, 8, 55, 56, 57, 86, 87, 88, 89, 90, 91), the clamping surfaces (27, 81) of said clamping elements lying on an outer diameter adjusted to the diameter of the axial bore (35, 35/1, 35/2) of the screw head of the bone screw (30, 30/1, 30/2) to achieve a clamping connection.

2. The screwdriver according to claim 1, **characterised in that** the clamping elements (20, 21, 22, 23) comprise circular sectors (27) of a central cylindrical pin (26), which are formed by at least two crossing, diametric grooves (24, 25, 125, 126) that are open to the front and radially, and **in that** the grooves (24, 25, 125, 126) penetrate into the shaft core (14) and have an axial depth, which is at least twice as large as the axial height of the clamping elements (5, 6, 7, 8, 127, 128, 129, 130), and **in that** the grooves (24, 25) lie respectively in the planes of symmetry (53 or 54) of two diametrically opposite sections (9, 11 or 10, 12).

3. The screwdriver according to claim 2, **characterised in that** fewer circular sectors (127, 128, 129, 130) are provided as tool blades (86, 87, 88, 89, 90, 91), wherein of the six tool blades (86, 87, 88, 89, 90, 91) two diametrically opposite tool blades (88, 91) have separate circular sectors (128, 130) on their front faces, and the front faces of two tool blades (86, 87 and 89, 90) lying in between in circumferential direction together have a circular sector (127, 129).

4. The screwdriver according to claim 1, **characterised in that** the clamping elements (66, 67, 68, 99, 100, 101, 102, 103, 104) consist of axially projecting sectors (20, 21, 22, 23) of a circular ring concentric to the axle (19) of the blade shaft (58, 85) and **in that** the sectors (66, 67, 68, 99, 100, 101, 102, 103, 104) of the circular ring are formed by sections (59, 60, 61, 105, 106, 107, 108, 109, 110), which form the tool blades (55, 56, 57, 86, 87, 88, 89, 90, 91) (Fig. 11).

5. The screwdriver according to claim 1, **characterised in that** the clamping elements consist of sectors (99, 100, 101, 102, 103, 104) of a circular ring lying on the front face (93, 94, 95, 96, 97, 98) of a tool blade (86, 87, 88, 89, 91) of a circular ring and are formed by radial grooves.

6. The screwdriver according to one of claims 1 to 5, **characterised in that** the clamping elements (66) have a peripheral surface (81) tapering conically to the plane of the front faces (63) of the tool blades (55) at an angle (β) of 2° to 6° and have a bevelled or rounded edge.

7. The screwdriver according to one of claims 2 or 3, **characterised in that** the radial grooves (24, 25, 125, 126) from the axially outer front faces of the tool blades (5, 6, 7, 8, 86, 87, 88, 89, 90, 91) have an axial depth of 0.5 to 2.5 mm.

## Revendications

1. Tournevis (1) pour vis à os (30, 30/1, 30/2), qui présente un outil doté d'au moins trois lames d'outil (5, 6, 7, 8, 55, 56, 57, 86, 87, 88, 89, 90, 91) s'étendant radialement et axialement, lesquelles peuvent être mises en prise par engagement positif avec des fentes de lame (36, 37, 38, 39, 70, 71, 72, 112, 113, 114, 115, 116, 117) de la vis à os qui sont semblables à des rainures et qui débouchent dans un perçage central cylindrique axial (35, 35/1, 35/2) de la vis à os (30, 30/1, 30/2), les lames d'outil (5, 6, 7, 8, 55, 56, 57, 86, 87, 88, 89, 90, 91) formant au moins un segment de serrage élastique (20, 21, 22, 23, 66, 67, 68, 99, 104, 127, 128, 129, 130) à l'aide duquel l'outil (4, 50, 51, 52) peut être mis en prise avec la vis à os (30, 30/1, 30/2) et immobilisé dans la zone du perçage axial (35, 35/1), **caractérisé en ce que** les segments de serrage des lames d'outil (5, 6, 7, 8, 55, 56, 57, 86, 87, 88, 89, 90, 91) sont chacun constitués d'éléments de serrage souples (20, 21, 22, 23, 66, 67, 68, 99, 100, 101, 102, 103, 104, 127, 128, 129, 130) faisant saillie des surfaces frontales (15, 16, 17, 18, 63, 64, 65, 93, 94, 95, 96, 97, 98) des lames d'outil (5, 6, 7, 8, 55, 56, 57, 86, 87, 88, 89, 90, 91), éléments de serrage dont les surfaces de serrage (27, 81) sont situées sur un diamètre extérieur adapté au diamètre du perçage axial (35, 35/1, 35/2) de la tête de la vis à os (30, 30/1, 30/2), afin de réaliser un assemblage par serrage.

2. Tournevis selon la revendication 1, **caractérisé en ce que** les éléments de serrage (20, 21, 22, 23) sont composés par les secteurs de cercle (27) d'un tourillon cylindrique central (26), qui sont formés par au moins deux rainures (24, 25, 125, 126) diamétrales ouvertes radialement sur leur face frontale, qui sont en intersection l'une avec l'autre, **en ce que** les rainures (24, 25, 125, 126) pénètrent dans le coeur de tige (14) et présentent une profondeur axiale qui équivaut au moins au double de la hauteur axiale des éléments de serrage (5, 6, 7, 8, 127, 128, 129, 130), et **en ce que** les rainures (24, 25) sont situées dans les plans de symétrie (respectivement 53 et 54) de deux découpes (respectivement 9, 11 et 10, 12) diamétralement opposées.

3. Tournevis selon la revendication 2, **caractérisé en ce qu'**il y a moins de secteurs de cercle (127, 128, 129, 130) que de lames d'outil (86, 87, 88, 89, 90, 91), sachant que, sur six lames d'outil (86, 87, 88, 89, 90, 91), deux lames d'outil (88, 91) diamétralement opposées présentent sur leurs surfaces frontales des secteurs de cercle séparés (128, 130), et que les surfaces frontales de deux lames d'outil (86, 87 et 89, 90) situées entre les précédentes portent ensemble un secteur de cercle (127, 129).

4. Tournevis selon la revendication 1, **caractérisé en ce que** les éléments de serrage (66, 67, 68, 99, 100, 101, 102, 103, 104) sont constitués par des secteurs (20, 21, 22, 23) d'un anneau concentrique à l'axe (19) de la tige de lame (58, 85), lesquels font saillie axialement, et **en ce que** les secteurs (66, 67, 68, 99, 100, 101, 102, 103, 104) de l'anneau sont formés par les découpes (59, 60, 61, 105, 106, 107, 108, 109, 110) qui forment les lames d'outil (55, 56, 57, 86, 87, 88, 89, 90, 91). (Fig. 11)

5. Tournevis selon la revendication 1, **caractérisé en ce que** les éléments de serrage sont constitués par des secteurs (99, 100, 101, 102, 103, 104) d'un anneau qui sont chaque fois situés sur la face frontale (93, 94, 95, 96, 97, 98) d'une lame d'outil (86, 87, 88, 89, 90, 91) et sont formés par des rainures s'étendant radialement.

6. Tournevis selon l'une des revendications 1 à 5, **caractérisé en ce que** les éléments de serrage (66) présentent une surface périphérique (81) se rétrécissant de façon conique avec un angle (β) compris entre 2° et 6°, en direction du plan des surfaces frontales (63) des lames d'outil (55), et une arête du bord chanfreinée ou arrondie.

7. Tournevis selon la revendication 2 ou 3, **caractérisé en ce que** les rainures s'étendant radialement (24, 25, 125, 126) présentent à partir des surfaces frontales extérieures des lames d'outil (5, 6, 7, 8, 86, 87, 88, 89, 90, 91) une profondeur axiale comprise entre 0,5 et 2,5 mm.
